# EUROPÄISCHE PATENTSCHRIFT

(11) **EP 3 553 548 B1**
(45) Veröffentlichungstag und Bekanntmachung des Hinweises auf die Patenterteilung: **08.06.2022**
(21) Anmeldenummer: 18167310.4
(22) Anmeldetag: 13.04.2018
(51) Int. Cl.: G01R 33/54, G01R 33/565, A61B 5/00, G05B 13/02, A61B 5/055

(54) **MAGNETRESONANZ-PULSSEQUENZEINSTELLUNG ANHAND BEWEGUNGSVORHERSAGE**
MAGNETIC RESONANCE PULSE SEQUENCE ADJUSTMENT USING MOTION FORECASTING
RÉGLAGE DE LA SÉQUENCE D'IMPULSIONS À RÉSONANCE MAGNÉTIQUE EN FONCTION D'UNE PRÉDICTION DU MOUVEMENT

(43) Veröffentlichungstag der Anmeldung: 16.10.2019
(73) Patentinhaber: Siemens Healthcare GmbH, 91052 Erlangen (DE)
(72) Erfinder: Speier, Peter, 91056 Erlangen (DE); Zeller, Mario, 91054 Erlangen (DE)

(56) Entgegenhaltungen:
- US-A1- 2010 145 182
- US-A1- 2015 212 182
- US-A1- 2016 000 383

## Beschreibung

Die Erfindung betrifft ein Verfahren zur Einstellung einer Pulssequenz einer MR-Untersuchung, eine MR-Vorrichtung und ein Computerprogrammprodukt.

In der Medizintechnik zeichnet sich die Bildgebung mittels Magnetresonanz (MR), auch Magnetresonanztomographie (MRT, engl. Magnetic Resonance Imaging, MRI) genannt, durch hohe und variable Weichteilkontraste aus. Hierbei werden mit Hilfe einer MR-Vorrichtung Anregungspulse einer Pulssequenz in einen Patienten eingestrahlt, welche im Patienten MR-Signale auslösen. Die MR-Signale werden üblicherweise durch MR-Spulen empfangen und an eine Auswerteeinheit weitergeleitet, um aus den MR-Signalen eine MR-Abbildung des Patienten zu erzeugen.

Bewegt sich der Patient bei der Aufnahme der MR-Signale, so kann dies die Qualität der MR-Abbildung negativ beeinflussen. Eine Patientenbewegung kann also die Diagnostik einschränken oder sogar gänzlich unmöglich machen. Unter Umständen kann eine Neuaufnahme der MR-Signale erforderlich sein.

Für dieses Problem existiert eine Vielzahl an Herangehensweisen. Dazu zählen insbesondere Korrekturmethoden, welche z.B. auf Navigatoren (siehe z.B. DE 102006055933 B4), externen Sensoren wie Kameras (siehe z.B. US 9714998 B2), Magnetfeldsonden (engl. magnetic field probes) oder Pilotton-Aufnahmen (engl. pilot tone) (siehe z.B. DE 102015203385 B4) basieren.

Weiterhin existieren Verfahren, bei denen Aufnahmen nur in bestimmen Atem- bzw. EKG-Zuständen (siehe z.B. US 20110130644 A1) durchgeführt werden. Die Festlegung der Aufnahmezeitpunkte geschieht dabei beispielsweise mittels externer Sensoren oder Navigatoren. Bei einem Navigator werden üblicherweise verschachtelt mit der eigentlichen Bildaufnahme Navigatordaten aus dem MR-Signal extrahiert (z.B. eine niedrig aufgelöste Darstellung des Kopfes).

Zusätzlich werden oftmals Verfahren verwendet, die inhärent robust gegenüber Bewegungen sind (beispielsweise radiale, insbesondere PROPELLER-Bildgebungs-Sequenzen) und/oder eine möglichst kurze Messzeit aufweisen und/oder mehrere Mittelungen umfassen, um den Einfluss von Bewegung zu reduzieren. KÜSTNER, Thomas et al. Automated reference-free detection of motion artifact in magnetic resonance images. In: Magn Reson Mater Phys, 2017. DOI 10.1007/s10334-017-0650-z, beschreibt ein bildbasiertes Deep-Learning-Verfahren, welches ermöglicht, retrospektiv aufgenommene Bilder hinsichtlich der aufgetretenen Bewegung zu beurteilen. Da das Verfahren jedoch bildbasiert arbeitet, steht das Ergebnis erst nach erfolgter Aufnahme fest, wodurch das Problem von Neuaufnahmen nicht vermieden wird.

US 2015/212182 offenbart ein Verfahren zur Einstellung einer Pulssequenz einer MR-Untersuchung eines Patienten, wobei anhand von Navigatordaten eine Matrix konstruiert wird, welche anschließend hinsichtlich vorkommender Bewegungen klassifiziert wird, um Entscheidungen über den weiteren Verlauf der MR-Untersuchung zu treffen.

US 2016/000383 offenbart ein Verfahren zur Einstellung einer Pulssequenz einer MR-Untersuchung eines Patienten basierend auf physiologischen Signalen.

US 2010/145182 offenbart ein Verfahren zur Einstellung einer Pulssequenz einer MR-Untersuchung eines Patienten basierend auf der Unruhe des Patienten.

Die Aufgabe der vorliegenden Erfindung ist, eine Pulssequenz so einzustellen, dass etwaige daraus resultierende MR-Abbildungen eine höhere Qualität aufweisen. Idealerweise sollen dadurch Neuaufnahmen vermieden werden.

Die Aufgabe wird durch die Merkmale der unabhängigen Ansprüche gelöst. Vorteilhafte Ausgestaltungen sind in den abhängigen Ansprüchen beschrieben.

Demnach wird ein Verfahren zur Einstellung einer Pulssequenz zur Bildgebung einer MR-Untersuchung eines Patienten gemäß Anspruch 1 vorgeschlagen, welches unter anderem folgende Schritte umfasst: . Es wird ein Untersuchungs-Bewegungssignal erfasst, das abhängig von der Bewegung des Patienten ist. Anhand des Untersuchungs-Bewegungssignals und einer Bestimmungsinformation wird eine Bewegungswahrscheinlichkeitsinformation, insbesondere eine Maßzahl, bestimmt. Die Bewegungswahrscheinlichkeitsinformation umfasst eine Wahrscheinlichkeitsangabe, insbesondere eine Vorhersage, zukünftiger Bewegung des Patienten. Unter einer zukünftigen Bewegung ist vorzugsweise eine Bewegung im weiteren Verlauf der MR-Untersuchung (z.B. innerhalb der folgenden 5 bis 50 Minuten), insbesondere während einer Erfassung von MR-Signalen, zu verstehen. Anhand der Bewegungswahrscheinlichkeitsinformation wird die Pulssequenz eingestellt.

Vorteilhafterweise wird die Bewegungswahrscheinlichkeitsinformation durch eine Recheneinheit ermittelt. Vorteilhafterweise wird die Pulssequenz durch eine Recheneinheit eingestellt.

Das Untersuchungs-Bewegungssignal ist ein Signal, dass die Bewegung des Patienten während zumindest eines Teils einer MR-Untersuchung in einem Zeitraum vor einer Erfassung von MR-Signalen beschreibt. Unter MR-Untersuchung ist hier also nicht nur der Zeitraum der Erfassung von MR-Signalen zu verstehen, sondern die MR-Untersuchung umfasst einen Zeitraum vor der Erfassung von MR-Signalen, insbesondere einen Zeitraum, in dem der Patient auf die Erfassung von MR-Signalen vorbereitet wird. Ein Teil einer MR-Untersuchung kann also beispielsweise auch der Zeitraum sein, in dem der Patient auf einer Lagerungsvorrichtung einer MR-Vorrichtung positioniert wird und/oder auch ein Zeitraum sein, in dem der Patient auf der Lagerungsvorrichtung verfahren wird.

Ein Teil der MR-Untersuchung kann auch eine Justagemessung sein. Die Justagemessung ist vorzugsweise eine Messung, die vor einer klinischen Messung, insbesondere vor einer Erfassung von MR-Signalen zur klinischen Diagnostik, stattfindet. Die Justagemessung umfasst vorzugsweise eine Adjustment-Prozedur. Die Justagemessung wird vorzugsweise ohne Zutun des Bedienpersonals ausgeführt. Als Justagemessung eignen sich insbesondere Messungen, die auf die Abbildung der relevanten Bewegung hin optimiert sind, z.B. Messungen mit hoher Bildwiederholrate, die die sich bewegenden Bereiche aufnehmen. Das Untersuchungs-Bewegungssignal ist abhängig von der Bewegung des Patienten. Das Untersuchungs-Bewegungssignal kann insbesondere ein physiologisches Untersuchungs-Bewegungssignal sein. Insbesondere umfasst das Untersuchungs-Bewegungssignal Informationen über die Bewegung des Patienten, beispielsweise Bewegungen des Kopfes, der Arme, der Beine, des Bauches und/oder der Brust des Patienten. Weitere mögliche Bewegungen sind Bewegungen innerer Organe, z.B. des Herzens und/oder peristaltische Bewegungen. Vorzugsweise beschreibt das Untersuchungs-Bewegungssignal eine Bewegung des Patienten, insbesondere eines Teils des Patienten. Vorzugsweise können aus dem Untersuchungs-Bewegungssignal Rückschlüsse über die Bewegung des Patienten gezogen werden.

Die Bestimmungsinformation umfasst vorzugsweise eine Information, die zur Bestimmung der Bewegungswahrscheinlichkeitsinformation aus dem Untersuchungs-Bewegungssignal verwendet werden kann. Vorzugsweise wird die Bestimmungsinformation abgeleitet aus zuvor erfassten MR-Signalen, insbesondere aus einer Bewertung zuvor erfasster MR-Signale. Die Bestimmungsinformation wird aus einer Kombination und/oder Verknüpfung, insbesondere einem Vergleich, des Untersuchungs-Bewegungssignals mit zuvor erfassten weiteren Bewegungssignalen abgeleitet.

Dabei sind die weiteren Bewegungssignale vorteilhafterweise den zuvor erfassten, insbesondere bewerteten, MR-Signalen zugeordnet. Somit kann vorteilhafterweise aus einer Bewertung des zuvor erfassten MR-Signals eine Relation zwischen einem Bewegungssignal und einer Bewegungswahrscheinlichkeitsinformation hergestellt werden.

Durch die Bestimmung der Bewegungswahrscheinlichkeitsinformation kann eine Vorhersage über die Wahrscheinlichkeit von Patientenbewegung getroffen werden. Diese Vorhersage kann bereits vor der Erfassung etwaiger MR-Signale, aus denen MR-Abbildungen erzeugt werden können, getroffen werden. Die Erfinder haben erkannt, dass bereits ein relativ kurzer Zeitraum der MR-Untersuchung ausreicht, den Patienten hinsichtlich seines Vermögens, etwaige Bewegungen zu unterdrücken, abzuschätzen.

Anhand der Bewegungswahrscheinlichkeitsinformation wird dann eine Pulssequenz eingestellt, die optimal zu dem Vermögen des Patienten, etwaige Bewegungen zu unterdrücken, passt. Für eine T2-gewichtete MR-Untersuchung wären beispielsweise je nach Bewegungswahrscheinlichkeitsinformation eine Entscheidung zwischen einer SPACE-, TSE-, PROPELLER- oder HASTE-Pulssequenz und/oder eine Entscheidung zwischen einer 3D- oder 2D-Multislice-Pulssequenz denkbar. Hier stehen SPACE für "Sampling Perfection with Application optimized Contrasts using different flip angle Evolutions", TSE für "Turbo Spin-Echo", PROPELLER für "Periodically Rotated Overlapping Parallel Lines with Enhanced Reconstruction" und HASTE für "Half-Fourier Acquisition Single-shot Turbo Spin-Echo".

Vorzugsweise wird mit der eingestellten Pulssequenz ein MR-Signal erfasst. Ein daraus resultierender Vorteil kann insbesondere darin bestehen, dass die erfassten MR-Signale von hoher Qualität sind.

Vorzugsweise wird anhand des erfassten MR-Signals eine MR-Abbildung erzeugt. Ein daraus resultierender Vorteil kann insbesondere darin bestehen, dass die erzeugte MR-Abbildung keine oder weniger Bewegungsartefakte aufweist.

Eine bevorzugte Ausführungsform des Verfahrens sieht vor, dass die Bewegungswahrscheinlichkeitsinformation eine Stärke und/oder Art zukünftiger Bewegung des Patienten umfasst. Ein daraus resultierender Vorteil liegt insbesondere darin, dass aus diesen Informationen die Pulssequenz noch präziser eingestellt werden kann.

Die Stärke der Bewegung kann beispielsweise eine Geschwindigkeit und/oder eine Amplitude einer Bewegung des Patienten umfassen. Die Art der Bewegung kann beispielsweise die Information umfassen, welcher Körperteil des Patienten die Bewegung ausführt oder in welche Richtung die Bewegung ausführt wird oder ob die Bewegung ein bestimmtes Muster (z.B. Kopfnicken) aufweist und/oder abrupt ist.

Eine bevorzugte Ausführungsform des Verfahrens sieht vor, dass das Untersuchungs-Bewegungssignal während einer Verweilzeit des Patienten auf einer Patientenlagerungsvorrichtung einer MR-Vorrichtung permanent erfasst wird. Vorzugsweise wird das Untersuchungs-Bewegungssignal während der gesamten Verweilzeit des Patienten auf einer Patientenlagerungsvorrichtung der MR-Vorrichtung permanent erfasst. Beispielsweise wird das Untersuchungs-Bewegungssignal fortwährend aufgezeichnet, solange der der Patient auf der Patientenlagerungsvorrichtung liegt.

Unter einer permanenten Erfassung kann hier eine Erfassung verstanden werden, bei der im Rahmen der technischen Möglichkeiten der Einheit, mit der das Untersuchungs-Bewegungssignal erfasst wird, das Untersuchungs-Bewegungssignal ununterbrochen erfasst wird. Ein daraus resultierender Vorteil liegt beispielsweise darin, dass dadurch eine große Datenmenge erzeugt wird, die wirkungsvoll zur Bestimmung der Bewegungswahrscheinlichkeitsinformation verwendet werden kann. Außerdem ist eine durchgehende Datenerfassung einfacher umzusetzen als beispielsweise eine irgendwie getriggerte Datenerfassung.

Eine bevorzugte Ausführungsform des Verfahrens sieht vor, dass die Bestimmung der Bewegungswahrscheinlichkeitsinformation und die Einstellung der Pulssequenz anhand der Bewegungswahrscheinlichkeitsinformation während der MR-Untersuchung, insbesondere während einer Erfassung von MR-Signalen, wiederholt ausgeführt werden. Beispielsweise wird die Pulssequenz während ihres Ablaufs anhand einer wiederholt ermittelten Bewegungswahrscheinlichkeitsinformation neu eingestellt. So kann vorteilhafterweise die Pulssequenz dynamisch angepasst werden.

Dies ist insbesondere dann von Vorteil, wenn der Patient im Laufe der MR-Untersuchung sein Bewegungsverhalten ändert.

Wenn beispielsweise der Patient unruhiger wird, so dass er sich stärker bewegt, kann eine bewegungsrobustere Pulssequenz eingestellt werden. Wenn beispielsweise der Patient ruhiger wird, so dass er sich weniger stark bewegt, kann eine Pulssequenz mit höherer räumlicher Auflösung eingestellt werden.

Vorzugsweise wird bei einer wiederholten Einstellung der Pulssequenz ein Mittelungsverfahren angepasst, insbesondere eine Mittelungszahl geändert, und/oder ein Teil der Pulssequenz neu aufgenommen. Dadurch können Bewegungsartefakte vermieden werden.

Unter einem Mittelungsverfahren kann hier insbesondere ein Verfahren verstanden werden, bei dem mehrere Messdaten, insbesondere eines zu erfassenden MR-Signals, gemittelt werden. Unter einer Mittelungszahl kann hier insbesondere eine Anzahl an Messdaten an einer gleichen k-Raum-Position verstanden werden, die gemittelt werden.

Eine bevorzugte Ausführungsform des Verfahrens sieht vor, dass das Untersuchungs-Bewegungssignal während einer Positionierung des Patienten auf einer Patientenlagerungsvorrichtung einer MR-Vorrichtung und/oder während einer Verschiebung der Patientenlagerungsvorrichtung erfasst wird. Die Untersuchungszeit kann damit optimal genutzt werden, so dass beispielsweise die MR-Untersuchung schneller durchgeführt werden kann.

Eine bevorzugte Ausführungsform des Verfahrens sieht vor, dass die Einstellung der Pulssequenz anhand der Bewegungswahrscheinlichkeitsinformation automatisiert und/oder manuell erfolgt.

Eine bevorzugte Ausführungsform des Verfahrens sieht vor, dass anhand der Bewegungswahrscheinlichkeitsinformation zumindest eine Pulssequenz vorgeschlagen wird, wobei aus der zumindest einen vorgeschlagenen Pulssequenz eine Pulssequenz ausgewählt und/oder bearbeitet wird.

Der Vorschlag wird vorteilhafterweise automatisch, insbesondere ohne Einwirkung des Bedienpersonals, durch eine Recheneinheit ermittelt. Vorzugsweise wird die zumindest eine Pulssequenz einem Bedienpersonal vorgeschlagen.

Insbesondere können die vorgeschlagene Pulssequenz oder die vorgeschlagenen Pulssequenzen auf einem Monitor angezeigt werden. Das Bedienpersonal kann beispielsweise den Vorschlag bestätigen und/oder korrigieren. Insbesondere kann das Bedienpersonal die zumindest eine vorgeschlagene Pulssequenz bestätigen oder ablehnen oder aus mehreren vorgeschlagenen Pulssequenzen eine Pulssequenz auswählen.

Das erfindungsgemäße Verfahren sieht vor, dass ein bekannter Anteil des Untersuchungs-Bewegungssignals aus dem Untersuchungs-Bewegungssignal abgetrennt wird und die Ermittlung der Bewegungswahrscheinlichkeitsinformation anhand des verbleibenden Untersuchungs-Bewegungssignals erfolgt. Der bekannte Anteil ist hierbei ein Anteil einer inneren Organbewegung, insbesondere eines Herzschlags und/oder einer peristaltischen Bewegung, und/oder einer Atmung. Beispielsweise kann bei einem Herzschlag nach Abtrennung eines regelmäßigen Anteils ein arrhythmischer Anteil als verbleibender Anteil extrahiert werden. Dies ermöglicht insbesondere eine besonders genaue Auswertung.

Eine bevorzugte Ausführungsform des Verfahrens sieht vor, dass die Bestimmungsinformation mit folgenden Schritten ermittelt wird: Ein Trainings-Bewegungssignal wird erfasst, das abhängig von der Bewegung des Patienten ist. Während der Erfassung des Trainings-Bewegungssignals wird ein Trainings-MR-Signal erfasst. Die Bestimmungsinformation wird durch Bewertung des Trainings-MR-Signals, insbesondere durch Bewertung einer aus dem Trainings-MR-Signal erzeugten Trainings-MR-Abbildung, ermittelt. Die Bestimmungsinformation wird vorzugsweise vor der Erfassung des Untersuchungs-Bewegungssignals ermittelt.

Dabei ist vorteilhafterweise die Bestimmungsinformation mit dem Trainings-Bewegungssignal verknüpft. Durch die Bewertung des Trainings-MR-Signals wird vorzugsweise auch das zugeordnete Trainings-Bewegungssignal bewertet, das während des Trainings-MR-Signals erfasst wurde. Somit kann eine Relation hergestellt werden zwischen der Bestimmungsinformation und dem Trainings-Bewegungssignal.

Vorzugsweise ist das Trainings-Bewegungssignal abhängig von der Bewegung des Patienten. Das Trainings-Bewegungssignal ist insbesondere ein physiologisches Untersuchungs-Bewegungssignal. Insbesondere umfasst das Trainings-Bewegungssignal Informationen über die Bewegung des Patienten. Vorzugsweise beschreibt das Trainings-Bewegungssignal eine Bewegung des Patienten, insbesondere eines Teils des Patienten. Vorzugsweise können aus dem Trainings-Bewegungssignal Rückschlüsse über die Bewegung des Patienten gezogen werden.

Eine bevorzugte Ausführungsform des Verfahrens sieht vor, dass das Trainings-Bewegungssignal in der gleichen Weise wie das Untersuchungs-Bewegungssignal erfasst wird. Insbesondere werden die gleiche Methode und/oder die gleichen Mittel, insbesondere ein gleicher Sensortyp, verwendet. Daraus kann insbesondere der Vorteil resultieren, dass das Trainings-Bewegungssignal und das Untersuchungs-Bewegungssignal leicht miteinander in Beziehung gesetzt, insbesondere verglichen, werden können.

Eine weitere Ausführungsform des Verfahrens sieht vor, dass das Trainings-Bewegungssignal von dem Trainings-MR-Signal verschieden ist. Insbesondere ist die Erfassungsweise des Trainings-Bewegungssignals von der Erfassungsweise des Trainings-MR-Signal verschieden. Insbesondere ist der Typ des Trainings-Bewegungssignals verschieden vom Typ des Trainings-MR-Signals. Insbesondere werden die verschiedene Methoden und/oder verschiedene Mittel verwendet. Insbesondere können spezielle physiologische Sensoren und/oder Verfahren, die geeignet sind die Bewegung des Patienten zu erfassen, zur Erfassung des Trainings-Bewegungssignals verwendet werden. Insbesondere kann das Untersuchungs-Bewegungssignal ungeeignet zur Erzeugung einer MR-Abbildung sein.

Hingegen ist das Trainings-MR-Signal vorzugsweise geeignet, daraus eine MR-Bildung zu erzeugen. Insbesondere wird das Trainings-MR-Signal durch eine MR-Spule erfasst, insbesondere durch eine MR-Lokalspule (engl. local coil) und/oder eine MR-Ganzkörperspule (engl. body coil).

Eine bevorzugte Ausführungsform des Verfahrens sieht vor, dass das Untersuchungs-Bewegungssignal und/oder das Trainings-Bewegungssignal mit zumindest einem Atemsensor und/oder einer Kamera und/oder zumindest einer Magnetfeldsonde und/oder mit einem Pilotton-Verfahren erfasst werden.

Der Atemsensor kann beispielsweise ein Atemgurt und/oder einer Spule sein, deren Beladung abhängig von einer Atembewegung ist.

Die Magnetfeldsonde misst vorzugsweise das Magnetfeld in einem Patientenaufnahmebereich, insbesondere einem Messbereich, insbesondere einem Sichtfeld (engl. field of view, FOV), der MR-Vorrichtung. Vorzugsweise sind als Magnetfeldsonden mehrere kleine Spulenelemente in MR-Spulen, insbesondere MR-Lokalspulen, verteilt. Sie können vorteilhafterweise zeitgleich und unabhängig von MR-Signalen angeregt und ausgelesen werden und liefern die Phase an ihrem jeweiligen Ort.

Bei dem Pilotton-Verfahren wird vorzugsweise ein schwaches und/oder kontinuierliches und/oder monofrequentes HF-Signal eingestrahlt. Das HF-Signal ist vorteilhafterweise abseits der Resonanzfrequenz der anzuregenden Substanz, deren Resonanz das MR-Signal auslöst. Das HF-Signal wird vorzugsweise in Amplitude und Phase durch Bewegung des Patienten in unterschiedlichen Elementen zumindest einer MR-Spule abhängig von deren Ort mit dem MR-Signal zusammen aufgenommen. Die Frequenz des HF-Signals ist vorteilhafterweise so gewählt, dass sie nicht mit dem MR-Signal überlappt, so dass es durch Frequenzanalyse von diesem separiert werden kann.

Eine bevorzugte Ausführungsform des Verfahrens sieht vor, dass die Ermittlung der Bestimmungsinformation anhand eines Ablaufs einer Trainings-MR-Untersuchung erfolgt, d.h. in die Ermittlung der Bestimmungsinformation geht der Ablauf der Trainings-MR-Untersuchung ein.

Insbesondere kann die Ermittlung der Bestimmungsinformation anhand von Messwiederholungen während der Trainings-MR-Untersuchung erfolgen. So kann beispielsweise aus einer Messwiederholung darauf geschlossen werden, dass die vorangehende Messung, insbesondere die vorangehend erfassten Trainings-MR-Signale, ungenügend war und das zugehörige Trainings-Bewegungssignal mit einer entsprechenden Information verknüpft werden. Mit einem derartigen Lernprozess kann die Bestimmungsinformation verbessert werden.

Eine bevorzugte Ausführungsform des Verfahrens sieht vor, dass alternativ zur Ermittlung der Bestimmungsinformation anhand eines Ablaufs einer Trainings-MR-Untersuchung oder damit kombiniert die Ermittlung der Bestimmungsinformation anhand einer visuellen Inspektion von aus den Trainings-MR-Signalen erzeugten Trainings-MR-Abbildungen erfolgt.

Beispielsweise kann ein Bedienpersonal einer MR-Vorrichtung aus Trainings-MR-Signalen erzeugte Trainings-MR-Abbildungen betrachten und diese bewerten. Weist eine Trainings-MR-Abbildung beispielsweise Artefakte auf, dann kann der Bediener die Trainings-MR-Abbildung entsprechend niedrig bewerten. Die niedrige Bewertung kann in Beziehung gesetzt werden zu dem Trainings-Bewegungssignal, das während der Erfassung des Trainings-MR-Signals erfasst wurde. Dieser Zusammenhang geht vorteilhafterweise in die Bestimmungsinformation ein. Bei einer MR-Untersuchung kann dann beispielsweise bei einem ähnlichen Untersuchungs-Bewegungssignal darauf geschlossen werden, dass eine erhöhte Wahrscheinlichkeit vorliegt, dass sich der Patient im weiteren Verlauf der MR-Untersuchung in ungünstiger Weise bewegt. Die Pulssequenz kann somit anhand der Bewegungswahrscheinlichkeitsinformation eingestellt werden, um Artefakte zu reduzieren.

Eine bevorzugte Ausführungsform des Verfahrens sieht vor, dass die Bestimmung einer Bewegungswahrscheinlichkeitsinformation und/oder die Ermittlung der Bestimmungsinformation anhand eines Algorithmus erfolgt, welcher auf maschinellem Lernen (engl. machine learning) und/oder einem künstlichen neuronalem Netz, insbesondere einem Convolutional Neural Network (CNN), basiert.

Insbesondere kann die Ermittlung der Bestimmungsinformation durch mit Hilfe eines automatisierten Bildbewertungsverfahrens erfolgen, insbesondere mit Verknüpfung einer daraus abgeleiteten Maßzahl. Ein solches automatisiertes Bildbewertungsverfahren ist beispielhaft in KÜSTNER, Thomas et al. Automated reference-free detection of motion artifact in magnetic resonance images. In: Magn Reson Mater Phys, 2017. DOI 10.1007/s10334-017-0650-z beschrieben. Eine Bewertung von Trainings-MR-Abbildungen, die aus den Trainings-MR-Signalen erzeugt wurden, erfolgt hier vorteilhafterweise automatisiert. Die weitere Verarbeitung der Bewertung kann insbesondere in gleicher Weise erfolgen, wie oben beschrieben bei einer visuellen Inspektion von aus den Trainings-MR-Signalen erzeugten Trainings-MR-Abbildungen.

Ein möglicher Vorteil eines automatisierten Verfahrens kann insbesondere darin bestehen, dass die Bestimmungsinformation ohne Benutzerinteraktion kontinuierlich verbessert werden kann. Somit kann die Bestimmungsinformation kontinuierlich trainiert werden.

Eine bevorzugte Ausführungsform des Verfahrens sieht vor, dass der Algorithmus, welcher auf maschinellem Lernen (engl. machine learning) und/oder einem künstlichen neuronalem Netz basiert, durch Trainings-MR-Signale und Trainings-Bewegungssignale verbessert wird, welche durch mehrere MR-Vorrichtungen erfasst werden. Somit können beispielsweise Trainingsdaten von dezentral, insbesondere weltweit, installierten MR-Vorrichtungen genutzt werden, um die Vorhersage der Bewegungswahrscheinlichkeit zu optimieren. Dazu sind die MR-Vorrichtungen vorteilhafterweise über ein Daten-Netzwerk miteinander und/oder mit einer Zentraleinheit verbunden.

Ferner wird eine MR-Vorrichtung vorgeschlagen, die ausgebildet ist, ein vorab beschriebenes Verfahren auszuführen. Die MR-Vorrichtung umfasst Mittel zur Erfassung eines Untersuchungs- und/oder Trainings-Bewegungssignals, zur Bestimmung einer Bewegungswahrscheinlichkeitsinformation und zur Einstellung der Pulssequenz.

Mittel zur Erfassung eines Untersuchungs- und/oder Trainings-Bewegungssignals können - wie vorab bereits erläutert - beispielsweise physiologische Sensoren, insbesondere ein Atemsensor, insbesondere ein Atemgurt, und/oder eine Kamera und/oder ein Magnetfeldsensor sein. Mittel zur Bestimmung einer Bewegungswahrscheinlichkeitsinformation und zur Einstellung der Pulssequenz können beispielsweise eine programmierbare Recheneinheit (z.B. mit Prozessoren, und/oder eine Speichereinheit) sein, die geeignet ist, ein Computerprogramm auszuführen.

Die Vorteile der erfindungsgemäßen MR-Vorrichtung entsprechen im Wesentlichen den Vorteilen des erfindungsgemäßen Verfahrens zur Einstellung einer Pulssequenz einer MR-Untersuchung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Ferner wird ein Computerprogrammprodukt vorgeschlagen, das ein Programm umfasst und direkt in einen Speicher einer programmierbaren Recheneinheit einer MR-Vorrichtung ladbar ist und Programmmittel, z.B. Bibliotheken und Hilfsfunktionen, aufweist, um ein vorab beschriebenes Verfahren zur Einstellung einer Pulssequenz auszuführen, wenn das Computerprogrammprodukt in der Recheneinheit ausgeführt wird. Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Überprüfungseinheit zu laden ist. Durch das Computerprogrammprodukt kann das Verfahren zur Einstellung einer Pulssequenz schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren durchführen. Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
Fig. 1 eine MR-Vorrichtung mit physiologischen Sensoren und einer Recheneinheit zur Einstellung einer Pulssequenz,
Fig. 2 ein Verfahren zur Einstellung einer Pulssequenz,
Fig. 3-4 erweiterte Verfahren zur Einstellung einer Pulssequenz,
Fig. 5-6 beispielhafte Verläufe von Bewegungssignalen,
Fig. 7 ein Ablauf einer MR-Untersuchung,
Fig. 8 mehrere MR-Vorrichtungen, die über ein Daten-Netzwerk direkt miteinander verbunden sind,
Fig. 9 mehrere MR-Vorrichtungen, die über ein Daten-Netzwerk mit einer Zentraleinheit miteinander verbunden sind.

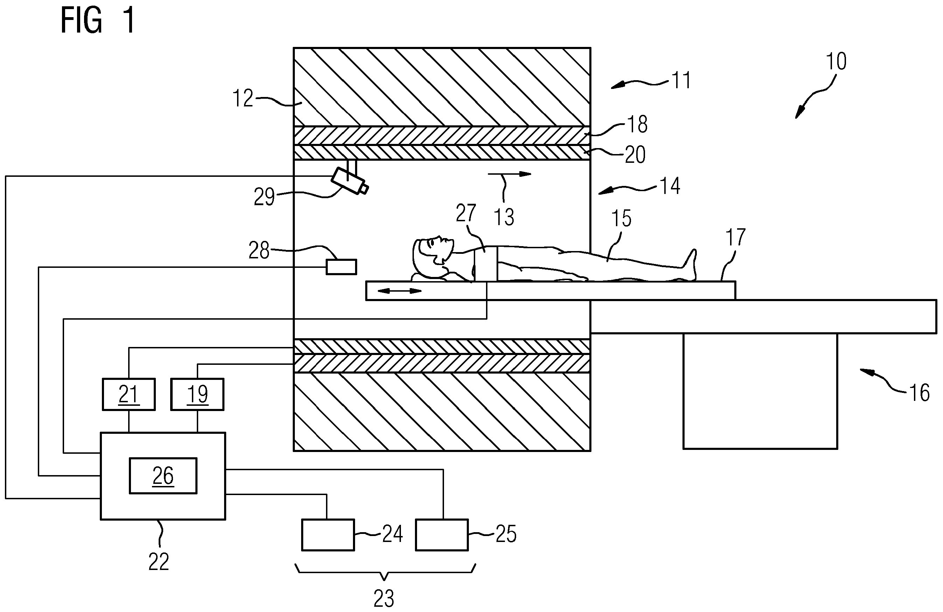

In Fig. 1 ist eine MR-Vorrichtung 10 schematisch dargestellt. Die MR-Vorrichtung 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die MR-Vorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der MR-Vorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der MR-Vorrichtung 10 gesteuert. Die Magneteinheit 11 umfasst weiterhin eine Antenneneinheit 20, welche im vorliegenden Ausführungsbeispiel als fest in die MR-Vorrichtung 10 integrierte MR-Ganzkörperspule ausgebildet ist. Die Antenneneinheit 20 ist zu einer Anregung von Atomkernen, die sich in dem von dem Hauptmagneten 12 erzeugten Hauptmagnetfeld 13 einstellt, ausgelegt. Die Antenneneinheit 20 wird von einer Antennensteuereinheit 21 der MR-Vorrichtung 10 gesteuert und strahlt eine Pulssequenz in einen Untersuchungsraum ein, der im Wesentlichen von einem Patientenaufnahmebereich 14 der MR-Vorrichtung 10 gebildet ist. Die Antenneneinheit 20 ist weiterhin zum Empfang von MR-Signalen ausgebildet.

Zu einer Steuerung des Hauptmagneten 12, der Gradientensteuereinheit 19 und zur Steuerung der Antennensteuereinheit 21 weist die MR-Vorrichtung 10 eine Systemsteuereinheit 22 auf. Die Systemsteuereinheit 22 steuert zentral die MR-Vorrichtung 10, wie beispielsweise das Durchführen einer bildgebenden Pulssequenz. Zudem umfasst die Systemsteuereinheit 22 eine nicht näher dargestellte Auswerteeinheit zu einer Auswertung von MR-Signalen, die während der MR-Untersuchung erfasst werden. Des Weiteren umfasst die MR-Vorrichtung 10 eine Benutzerschnittstelle 23, die mit der Systemsteuereinheit 22 verbunden ist. Steuerinformationen wie beispielsweise Bildgebungsparameter, sowie erzeugte MR-Abbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Ferner umfasst die MR-Vorrichtung 10 beispielhaft mehrere physiologische Sensoren wie einem Atemgurt 27, eine Magnetfeldsonde 28 und eine Kamera 29, die mit der Systemsteuereinheit verbunden sind. Mit den Sensoren 27, 28, 29 können Bewegungssignale, insbesondere ein Untersuchungs-Bewegungssignal und/oder ein Trainings-Bewegungssignal, erfasst werden.

Eine solche Vielzahl an Sensoren 27, 28, 29, wie hier dargestellt, ist jedoch nicht notwendig, d.h. auch beispielsweise nur ein Sensor kann ausreichend sein. Möglicherweise kann auch ganz auf spezielle Sensoren verzichtet werden, solange es in irgendeiner Weise möglich ist, ein Bewegungssignal des Patienten 15 zu erfassen.

Ferner kann die MR-Vorrichtung 10 einen oder mehrere hier nicht dargestellte Sender zur Erzeugung eines HF-Signals umfassen, mit dem ein Pilotton-Verfahren ausgeführt werden kann. Mit dem Pilotton-Verfahren können auch Bewegungssignale des Patienten 15 erfasst werden.

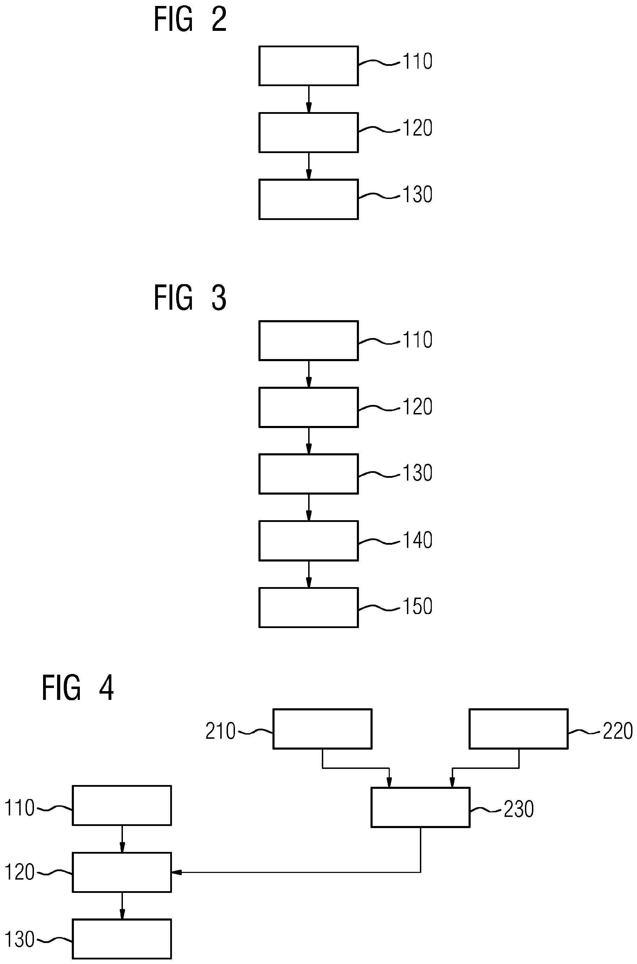

Ferner umfasst die Systemsteuereinheit 22 eine programmierbare Recheneinheit 26 mit einem Speicher, in das ein Computerprogrammprodukt geladen werden kann, um ein in Fig. 2-4 dargestelltes Verfahren auszuführen.

Gemäß Fig. 2 wird in einem Schritt 110 ein Untersuchungs-Bewegungssignal erfasst, das abhängig von der Bewegung des Patienten ist. Diese können beispielsweise mit den bereits beschriebenen Sensoren 27, 28, 29 und/oder mit einem Pilotton-Verfahren erfasst werden. Ferner ist z.B. auch eine kontinuierliche Sequenz-Navigator-Aufnahme oder/oder eine Bewegungserfassung während einer Justagemessung denkbar.

Eine Justagemessung ist vorzugsweise eine Messung, die ohne Zutun des Bedienpersonals als Teil einer der klinischen Messung vorgeschalteten Adjustment-Prozedur gestartet wird. Als Justagemessung eignen sich insbesondere Messungen, die auf die Abbildung der relevanten Bewegung hin optimiert sind, z.B. Messungen mit hoher Bildwiederholrate, die die sich bewegenden Bereiche aufnehmen.

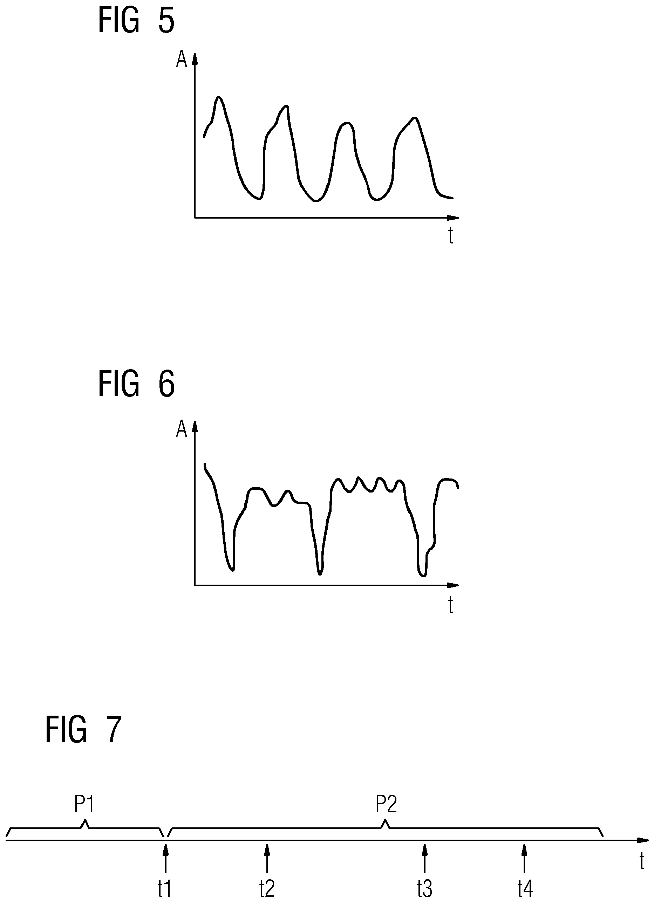

In Fig. 5-6 sind beispielhafte Verläufe von Bewegungssignalen dargestellt, bei denen eine Signalamplitude A über die Zeit t aufgetragen sind. In Fig. 5 wird ein Atemsignal und in Fig. 6 eine Handbewegung dargestellt. Man erkennt, dass aus den Bewegungssignalen Informationen über die Bewegung, insbesondere über die Art und Stärke der Bewegung abgeleitet werden können.

Die Bewegungssignale werden bevorzugt während der Verweilzeit des Patienten 15 auf dem Patiententisch 17 permanent aufgezeichnet. Je nach Sensortyp kann dies bereits bei der Positionierung des Patienten 15 auf dem Patiententisch 17, während eines Verfahrens des Patiententisches 17 und/oder sobald der Patient 15 in das Isozentrum (üblicherweise die Mitte des Patientenaufnahmebereichs 14) der MR-Vorrichtung 10 gefahren wurde, erfolgen.

In Schritt 120 der Fig. 2 wird eine Bewegungswahrscheinlichkeitsinformation anhand des Untersuchungs-Bewegungssignals und einer Bestimmungsinformation erfasst. Dabei umfasst die Bewegungswahrscheinlichkeitsinformation eine Wahrscheinlichkeitsangabe zukünftiger Bewegung des Patienten 15.

Beispielsweise wird vor jeder geplanten Bildgebungssequenz eine aktuelle Wahrscheinlichkeitsangabe in Form einer Maßzahl ermittelt. Diese Maßzahl kann dem Bedienpersonal beispielsweise direkt auf der Anzeigeeinheit 24 angezeigt werden oder automatisch für die Wahl einer optimalen Untersuchungsstrategie für die nächste Pulssequenz verwendet werden.

Alternativ zur Maßzahl kann ein Algorithmus, welcher auf maschinellem Lernen und/oder einem künstlichen neuronalem Netz, insbesondere einem Convolutional Neural Network (CNN), basiert, auch direkt die günstigste Untersuchungsmethode in Form einer Pulssequenz für die jeweils gefundene Bewegungsanfälligkeit zurückliefern. Für eine T2-gewichtete MR-Untersuchung wären beispielsweise je nach Bewegungswahrscheinlichkeitsinformation eine Entscheidung zwischen einer SPACE-, TSE-, PROPELLER- oder HASTE-Pulssequenz und/oder eine Entscheidung zwischen einer 3D- oder 2D-Multislice-Pulssequenz denkbar. Die Einstellung der Pulssequenz stellt dann Schritt 130 dar.

Das Verfahren gemäß Fig. 3 ist dahingehend erweitert, dass zusätzlich in Schritt 140 MR-Signale erfasst werden. Das MR-Signal wird mittels der MR-Vorrichtung 10 durch Einstrahlung der eingestellten Pulssequenz wie vorab erklärt aufgenommen.

In Schritt 150 wird anhand der erfassten MR-Signale eine MR-Abbildung erzeugt. Diese können beispielsweise auf der Anzeigeeinheit 24 angezeigt werden.

In Fig. 4 wird ferner dargestellt, wie die Bestimmungsinformation ermittelt werden kann, insbesondere der besagte Algorithmus trainiert werden kann. In Schritt 210 wird ein Trainings-Bewegungssignal erfasst, das wie das Untersuchungs-Bewegungssignal abhängig von der Bewegung des Patienten ist. Hier können auch wieder die Sensoren 27, 28, 29 und/oder Verfahren eingesetzt werden, die auch für die Erfassung des Untersuchungs-Bewegungssignals eingesetzt werden. Insbesondere kann das Untersuchungs-Bewegungssignal in der gleichen Weise wie das Trainings-Bewegungssignal erfasst werden.

Während der Erfassung des Trainings-Bewegungssignals in Schritt 210 erfolgt in Schritt 220 die Erfassung eines Trainings-MR-Signals. Das Trainings-MR-Signal ist ein bildgebendes MR-Signal, wie es bereits vorab beschrieben wurde.

Vorzugsweise werden für das Training des besagten Algorithmus die Schritte 210 und 220 nicht nur einmal durchgeführt, sondern es wird eine Vielzahl an MR-Aufnahmen durchgeführt.

In Schritt 230 wird die Bestimmungsinformation durch Bewertung des Trainings-MR-Signal ermittelt. Dies kann manuell und/oder automatisiert erfolgen.

Ein manuelles Verfahren kann z.B. auf einer manuellen Bewertung nach einer retrospektiven visuellen Inspektion von MR-Abbildungen basieren, die aus den Trainings-MR-Signalen erzeugt wurden. Diese Bewertung und das in Schritt 210 erfasste Trainings-Bewegungssignal gehen in die Ermittlung der Bestimmungsinformation ein, d.h. der besagte Algorithmus wird dadurch trainiert.

Ein automatisches Training kann beispielsweise anhand eines Algorithmus erfolgen, das auf maschinellem Lernen und/oder einem künstlichen neuronalem Netz, insbesondere einem Convolutional Neural Network (CNN) beruht. Insbesondere kann das Training durch eine Verknüpfung mit einer Bewertung einer MR-Abbildung erfolgen, die mit einem Automatisierungen Bildbewertungsverfahren bestimmt wird, wie es beispielhaft in KÜSTNER, Thomas et al. Automated reference-free detection of motion artifact in magnetic resonance images. In: Magn Reson Mater Phys, 2017. DOI 10.1007/s10334-017-0650-z beschrieben ist. Vorteilhaft ist hierbei, dass zum Training keine Interaktion des Bedienpersonals erforderlich ist.

Bei beiden Verfahren werden die Trainings-Bewegungssignale während der Aufnahme der bildgebenden Trainings-MR-Signale aufgezeichnet, so dass diese Daten miteinander verknüpft werden können. Dadurch kann in beiden Fällen ein retrospektives Training erfolgen, was die Genauigkeit des Verfahrens erhöht.

Weiterhin kann auf Basis von erkannten Messwiederholungen, die durch das Bedienpersonal durchgeführt wurden, eine Bewertung des Trainings-MR-Signals abgeleitet werden.

Ferner kann die Bewegungswahrscheinlichkeitsinformation, insbesondere eine Vorhersage, ob und/oder wie sich der Patient 15 bewegen wird, auch in kürzeren Zeitabständen während der Laufzeit der Pulssequenz erfolgen, um eine dynamische Anpassung der Parameter der Pulssequenz zu ermöglichen, z.B. eine Veränderung eine Mittelungszahl und/oder eine Neuaufnahme einzelner Sequenzteile. Dies wird beispielhaft in Fig. 7 dargestellt.

Zum einen kann schon während der Positionierung des Patienten 15 auf dem Patiententisch 17 im Zeitraum P1 die Einstellung der Pulssequenz durch die Schritte 110, 120 und 130 erfolgen, so dass zum Zeitpunkt t1 des Beginns der Laufzeit der Pulssequenz im Zeitraum P2 die Pulssequenz eingestellt wird.

Aber auch während der Laufzeit der Pulssequenz, also wenn bereits Bilddaten aufgenommen werden, kann die Pulssequenz wiederholt eingestellt werden, beispielsweise in den Zeitpunkten t2, t3 und t4. Die Bestimmung der Bewegungswahrscheinlichkeitsinformation und die Einstellung der Pulssequenz anhand der Bewegungswahrscheinlichkeitsinformation während der MR-Untersuchung werden hier also wiederholt ausgeführt.

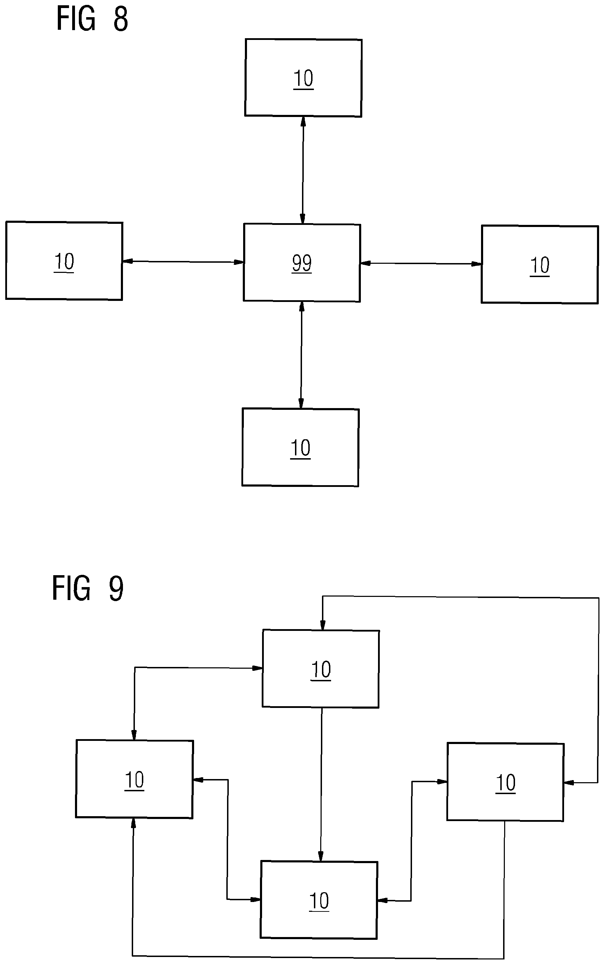

In Fig. 8 und 9 sind MR-Vorrichtungen 10 dargestellt, die über ein Daten-Netzwerk miteinander und/oder mit einer Zentraleinheit 99 verbunden sind. Somit können mehrere MR-Vorrichtungen 10 dazu beitragen, einen Algorithmus zur Bestimmung einer Bewegungswahrscheinlichkeitsinformation und/oder zur Ermittlung der Bestimmungsinformation zu verbessern. Insbesondere kann ein künstliches neuronales durch Trainings-MR-Signale und Trainings-Bewegungssignale trainiert werden.

Es wird abschließend noch einmal darauf hingewiesen, dass es sich bei den vorhergehend detailliert beschriebenen Verfahren sowie bei der dargestellten Erfassungsmustererzeugungseinheit und MR-Vorrichtung lediglich um Ausführungsbeispiele handelt, welche vom Fachmann in verschiedenster Weise modifiziert werden können, ohne den Bereich der Erfindung zu verlassen, welcher durch die Ansprüche definiert ist. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können. Erfolgt ein Verfahrensschritt "anhand" eines Mittels, so schließt dies nicht aus, dass noch weitere Mittel zum Verfahrensschritt beitragen.

## Patentansprüche

1. Verfahren zur Einstellung einer Pulssequenz zur Bildgebung im Rahmen einer MR-Untersuchung eines Patienten mit folgenden Schritten:
- Erfassung eines Untersuchungs-Bewegungssignals, das abhängig von der Bewegung des Patienten ist, in einem Zeitraum vor einer Erfassung von MR-Signalen während der MR-Untersuchung,
- Bestimmung einer Bewegungswahrscheinlichkeitsinformation anhand des Untersuchungs-Bewegungssignals und einer Bestimmungsinformation, wobei die Bewegungswahrscheinlichkeitsinformation eine Wahrscheinlichkeitsangabe zukünftiger Bewegung des Patienten umfasst,
wobei die Bestimmungsinformation aus einer Kombination und/oder Verknüpfung des Untersuchungs-Bewegungssignals mit zuvor erfassten weiteren Bewegungssignalen abgeleitet ist, wobei ein bekannter Anteil einer inneren Organbewegung und/oder einer Atmung des Untersuchungs-Bewegungssignals aus dem Untersuchungs-Bewegungssignal abgetrennt wird und die Ermittlung der Bewegungswahrscheinlichkeitsinformation anhand des verbleibenden Untersuchungs-Bewegungssignals erfolgt,
- Einstellung einer Pulssequenz, die optimal zu dem Vermögen des Patienten passt, etwaige Bewegungen zu unterdrücken, anhand der Bewegungswahrscheinlichkeitsinformation.

2. Verfahren nach Anspruch 1,
wobei die Bewegungswahrscheinlichkeitsinformation eine Stärke und/oder Art der zukünftigen Bewegung des Patienten umfasst.

3. Verfahren nach einem der vorangehenden Ansprüche,
wobei das Untersuchungs-Bewegungssignal während einer Verweilzeit des Patienten auf einer Patientenlagerungsvorrichtung einer MR-Vorrichtung permanent erfasst wird.

4. Verfahren nach einem der vorangehenden Ansprüche,
wobei die Bestimmung der Bewegungswahrscheinlichkeitsinformation und die Einstellung der Pulssequenz anhand der Bewegungswahrscheinlichkeitsinformation während der MR-Untersuchung wiederholt ausgeführt werden.

5. Verfahren nach Anspruch 4,
wobei bei einer wiederholten Einstellung der Pulssequenz eine Mittelungszahl geändert und/oder ein Teil der Pulssequenz neu aufgenommen wird.

6. Verfahren nach einem der vorangehenden Ansprüche,
wobei das Untersuchungs-Bewegungssignal erfasst wird
- während einer Positionierung des Patienten auf einer Patientenlagerungsvorrichtung einer MR-Vorrichtung und/oder
- während einer Verschiebung der Patientenlagerungsvorrichtung und/oder
- sobald sich der Patient in einer Messposition befindet, in der sich der Patient während einer Erfassung von MR-Signalen befindet.

7. Verfahren nach einem der vorangehenden Ansprüche,
wobei die Einstellung der Pulssequenz anhand der Bewegungswahrscheinlichkeitsinformation automatisiert und/oder manuell erfolgt.

8. Verfahren nach einem der vorangehenden Ansprüche,
wobei anhand der Bewegungswahrscheinlichkeitsinformation zumindest eine Pulssequenz automatisch einem Bedienpersonal vorgeschlagen wird,
wobei durch das Bedienpersonal aus der zumindest einen vorgeschlagenen Pulssequenz eine Pulssequenz ausgewählt und/oder bearbeitet wird.

9. Verfahren nach einem der vorangehenden Ansprüche,
wobei das Untersuchungs-Bewegungssignal mit zumindest einem Atemsensor und/oder einer Kamera und/oder zumindest einem Magnetfeldsensor und/oder mit einem Pilotton-Verfahren erfasst werden.

10. Verfahren nach einem der vorangehenden Ansprüche,
wobei die Bestimmungsinformation mit folgenden Schritten ermittelt wird:
- Erfassung eines Trainings-Bewegungssignals, das abhängig von der Bewegung des Patienten ist,
- Erfassung eines Trainings-MR-Signals während der Erfassung des Trainings-Bewegungssignals,
- Ermittlung der Bestimmungsinformation durch Bewertung des Trainings-MR-Signals, wodurch durch die Bewertung des Trainings-MR-Signals auch das Trainings-Bewegungssignal bewertet wird, so dass eine Relation zwischen der Bestimmungsinformation und dem Trainings-Bewegungssignal hergestellt wird.

11. Verfahren nach Anspruch 10,
wobei das Untersuchungs-Bewegungssignal in einer gleichen Weise wie das Trainings-Bewegungssignal erfasst wird.

12. Verfahren nach einem der Ansprüche 10 oder 11,
wobei die Erfassungsweise des Trainings-Bewegungssignals von der des Trainings-MR-Signals verschieden ist.

13. Verfahren nach einem der Ansprüche 10 bis 12,
wobei das Trainings-Bewegungssignal mit zumindest einem Atemsensor und/oder einer Kamera und/oder zumindest einem Magnetfeldsensor und/oder mit einem Pilotton-Verfahren erfasst werden.

14. Verfahren nach einem der Ansprüche 10 bis 13,
wobei die Ermittlung der Bestimmungsinformation anhand einer visuellen Inspektion von aus den Trainings-MR-Signalen erzeugten Trainings-MR-Abbildungen durch ein Bedienpersonal erfolgt.

15. Verfahren nach einem der vorangehenden Ansprüche,
wobei die Bestimmung einer Bewegungswahrscheinlichkeitsinformation und/oder die Ermittlung der Bestimmungsinformation anhand eines Algorithmus erfolgt, welcher auf maschinellem Lernen (engl. machine learning) und/oder einem künstlichen neuronalem Netz, insbesondere einem Convolutional Neural Network (CNN), basiert.

16. Verfahren nach Anspruch 15,
wobei der Algorithmus durch Trainings-MR-Signale und/oder Trainings-Bewegungssignale verbessert wird, welche durch mehrere MR-Vorrichtungen erfasst werden.

17. MR-Vorrichtung, welche ausgebildet ist, ein Verfahren nach einem der Ansprüche 1 bis 16 durchzuführen,
wobei die MR-Vorrichtung Mittel zur Erfassung eines Untersuchungs-Bewegungssignals, zur Bestimmung einer Bewegungswahrscheinlichkeitsinformation und zur Einstellung der Pulssequenz umfasst, wobei, wenn die MR-Vorrichtung zur Durchführung eines Verfahrens nach einem der Ansprüche 10 bis 14, oder nach einem der Ansprüche 15 oder 16, falls diese von einem der Ansprüche 10 bis 14 abhängig sind, ausgebildet ist, die MR-Vorrichtung zusätzlich Mittel zur Erfassung eines Trainings-Bewegungssignals umfasst.

18. Computerprogrammprodukt, welches ein Programm umfasst und direkt in einen Speicher einer programmierbaren Recheneinheit einer MR-Vorrichtung ladbar ist, mit Programmmitteln, welche ausgebildet sind, ein Verfahren nach einem der Ansprüche 1 bis 16 auszuführen, wenn das Programm in der Recheneinheit ausgeführt wird,
wobei die MR-Vorrichtung Mittel zur Erfassung eines Untersuchungs-Bewegungssignals, zur Bestimmung einer Bewegungswahrscheinlichkeitsinformation und zur Einstellung der Pulssequenz umfasst, wobei, wenn die Programmmittel zur Durchführung eines Verfahrens nach einem der Ansprüche 10 bis 14, oder nach einem der Ansprüche 15 oder 16, falls diese von einem der Ansprüche 10 bis 14 abhängig sind, ausgebildet sind, die MR-Vorrichtung zusätzlich Mittel zur Erfassung eines Trainings-Bewegungssignals umfasst.

## Claims

1. Method for adjusting a pulse sequence for imaging in the context of an MR examination of a patient having the following steps:
- detecting an examination movement signal, which is dependent on the movement of the patient, in a period before detecting MR signals during the MR examination,
- determining an item of movement probability information using the examination movement signal and an item of determination information, wherein the item of movement probability information comprises a probability detail relating to the future movement of the patient,
wherein the item of determination information is derived from a combination and/or linking of the examination movement signal with previously detected further movement signals, wherein a known portion of an inner organ movement and/or a breathing of the examination movement signal is separated from the examination movement signal and the determination of the movement probability information is carried out using the remaining examination movement signal,
- adjusting a pulse sequence, which is optimally matched to the capability of the patient to suppress possible movements, using the item of movement probability information.

2. Method according to claim 1,
wherein the item of movement probability information comprises a strength and/or type of future movement of the patient.

3. Method according to one of the preceding claims,
wherein the examination movement signal is detected permanently during a dwell time of the patient on a patient support apparatus of an MR apparatus.

4. Method according to one of the preceding claims,
wherein the determination of the item of movement probability information and the adjustment of the pulse sequence are carried out repeatedly during the MR examination using the item of movement probability information.

5. Method according to claim 4,
wherein with a repeated adjustment of the pulse sequence, an averaging number is changed and/or a part of the pulse sequence is recorded again.

6. Method according to one of the preceding claims,
wherein the examination movement signal is detected
- during a positioning of the patient on a patient support apparatus of an MR apparatus and/or
- during a displacement of the patient support apparatus and/or
- once the patient is located in a measuring position, in which the patient is located during a detection of MR signals.

7. Method according to one of the preceding claims,
wherein the adjustment of the pulse sequence is carried out automatically and/or manually using the item of movement probability information.

8. Method according to one of the preceding claims,
wherein a pulse sequence is automatically proposed to an operating personnel using the item of movement probability information,
wherein a pulse sequence is selected and/or processed from the at least one proposed pulse sequence by the operating personnel.

9. Method according to one of the preceding claims,
wherein the examination movement signal is detected with at least one breathing sensor and/or a camera and/or at least one magnetic field sensor and/or with a pilot tone method.

10. Method according to one of the preceding claims,
wherein the item of determination information is determined with the following steps:
- detecting a training movement signal, which is dependent on the movement of the patient,
- detecting a training MR signal during the detection of the training movement signal,
- determining the item of determination information by evaluating the training MR signal, as a result of which the training movement signal is also evaluated by evaluating the training MR signal so that a relation between the item of determination information and the training movement signal is established.

11. Method according to claim 10,
wherein the examination movement signal is detected in a similar way to the training movement signal.

12. Method according to one of claims 10 or 11, wherein the method of detection of the training movement signal differs from that of the training MR signal.

13. Method according to one of claims 10 to 12,
wherein the training movement signal is detected with at least one breathing sensor and/or a camera and/or at least one magnetic field sensor and/or with a pilot tone method.

14. Method according to one of claims 10 to 13,
wherein the determination of the determination information is carried out using a visual inspection of training MR images produced from the training MR signals by operating personnel.

15. Method according to one of the preceding claims,
wherein the determination of an item of movement probability information and/or the determination of the item of determination information is carried out using an algorithm which is based on machine learning and/or on an artificial neural network, in particular a convolutional neural network (CNN).

16. Method according to claim 15,
wherein the algorithm is improved by training MR signals and/or training movement signals which are detected by a number of MR apparatuses.

17. MR device, which is embodied to carry out a method according to one of claims 1 to 16, wherein the MR apparatus comprises means for detecting an examination movement signal for determining an item of movement probability information and for adjusting the pulse sequence, wherein if the MR apparatus is embodied to carry out a method according to one of claims 10 to 14, or according to one of claims 15 or 16, if these are dependent on one of claims 10 to 14, the MR apparatus additionally comprises means for detecting a training movement signal.

18. Computer program product, which comprises a program and can be loaded directly into a memory of a programmable computing unit of an MR apparatus, having program means which are embodied to carry out a method according to one of claims 1 to 16, if the program is executed in the computing unit, wherein the MR apparatus comprises means for detecting an examination movement signal, for determining an item of movement probability information and for adjusting the pulse sequence, wherein if the program means are embodied to carry out a method according to one of claims 10 to 14, or according to one of claims 15 or 16, if these depend on one of claims 10 to 14, the MR apparatus additionally comprises means for detecting a training movement signal.

## Revendications

1. Procédé de réglage d'une séquence d'impulsions pour l'imagerie, dans le cadre d'un examen RM d'un patient, comprenant les stades suivants :
- détection d'un signal de mouvement-d'examen, qui dépend du mouvement du patient, dans un laps de temps avant une détection de signaux RM pendant l'examen RM,
- détermination d'une information de probabilité de mouvement, à l'aide du signal de mouvement-d'examen et d'une information de détermination, l'information de probabilité de mouvement comprenant une indication de probabilité de mouvement à venir du patient,
dans lequel l'information de détermination est déduite d'une combinaison et/ou d'une réunion du signal de mouvement-d'examen a d'autres signaux de mouvement détectés auparavant, dans lequel on sépare, du signal de mouvement-d'examen, une proportion connue d'un mouvement intérieur d'organe et/ou d'une respiration du signal de mouvement-d'examen et la détermination de l'information de probabilité de mouvement s'effectue à l'aide du signal restant de mouvement-d'examen,
- réglage d'une séquence d'impulsions, qui s'adapte au mieux à la capacité du patient de supprimer d'éventuels mouvements, à l'aide de l'information de probabilité de mouvement.

2. Procédé suivant la revendication 1,
dans lequel l'information de probabilité de mouvement comprend une intensité et/ou un type du mouvement à venir du patient.

3. Procédé suivant l'une des revendications précédentes,
dans lequel on détecte en permanence le signal de mouvementd'examen, pendant un temps de séjour du patient sur une installation sur laquelle le patient est couché, d'une installation RM.

4. Procédé suivant l'une des revendications précédentes,
dans lequel on effectue, d'une manière répétée pendant l'examen RM, la détermination de l'information de probabilité de mouvement et le réglage de la séquence d'impulsions à l'aide de l'information de probabilité de mouvement.

5. Procédé suivant la revendication 4,
dans lequel, lors d'un réglage répété de la séquence d'impulsions, on modifie une moyenne et/ou on enregistre à nouveau une partie de la séquence d'impulsions.

6. Procédé suivant l'une des revendications précédentes,
dans lequel on détecte le signal de mouvement-d'examen
- pendant une mise en position du patient sur une installation, sur laquelle le patient est couché, d'une installation RM et/ou
- pendant un déplacement de l'installation, sur laquelle le patient est couché, et/ou
- dès que le patient se trouve dans une position de mesure, dans laquelle le patient se trouve pendant une détection de signaux RM.

7. Procédé suivant l'une des revendications précédentes,
dans lequel le réglage de la séquence d'impulsions, à l'aide de l'information de probabilité de mouvement, s'effectue d'une manière automatisée et/ou manuelle.

8. Procédé suivant l'une des revendications précédentes,
dans lequel, à l'aide de l'information de probabilité de mouvement, il est proposé automatiquement au moins une séquence d'impulsions à un personnel de service, dans lequel une séquence d'impulsions est choisie et/ou traitée par le personnel de service, à partir de la au moins une séquence d'impulsions proposée.

9. Procédé suivant l'une des revendications précédentes,
dans lequel on détecte le signal de mouvement-d'examen par au moins un capteur de respiration et/ou une caméra et/ou au moins un capteur de champ magnétique et/ou par un procédé à signal pilote.

10. Procédé suivant l'une des revendications précédentes,
dans lequel on détermine l'information de détermination par les stades suivants :
- détection d'un signal de mouvement-d'apprentissage, qui dépend du mouvement du patient,
- détection d'un signal RM-d'apprentissage pendant la détection du signal de mouvement d'apprentissage,
- détermination de l'information de détermination par évaluation du signal RM-d'apprentissage, grâce à quoi on évalue, par l'évaluation du signal RM-d'apprentissage, également le signal de mouvement d'apprentissage, de manière à produire une relation entre l'information de détermination et le signal de mouvement d'apprentissage.

11. Procédé suivant la revendication 10,
dans lequel on détecte le signal de mouvement-d'examen de la même façon que le signal de mouvement-d'apprentissage.

12. Procédé suivant l'une des revendications 10 ou 11,
dans lequel la façon de détecter le signal de mouvementd'apprentissage est différente de celle du signal RMd'apprentissage.

13. Procédé suivant l'une des revendications 10 à 12,
dans lequel on détecte le signal de mouvement-d'apprentissage par au moins un capteur de respiration et/ou une caméra et/ou au moins un capteur de champ magnétique et/ou par un procédé à signal pilote.

14. Procédé suivant l'une des revendications 10 à 13,
dans lequel la détermination de l'information de détermination s'effectue par un personnel de service à l'aide d'une inspection visuelle de reproductions-RM-d'apprentissage produite à partir des signaux-RM-d'apprentissage.

15. Procédé suivant l'une des revendications précédentes,
dans lequel la détermination d'une information de probabilité de mouvement et/ou la détermination de l'information de détermination s'effectue à l'aide d'un algorithme, qui repose sur un apprentissage automatique (en anglais machine learning) et/ou un réseau neuronal artificiel, notamment un réseau neuronal de convolution (CNN).

16. Procédé suivant la revendication 15,
dans lequel on améliore l'algorithme par des signaux RMd'apprentissage et/ou par des signaux de mouvement-d'apprentissage que l'on détecte par plusieurs installations RM.

17. Installation-RM, qui est constituée pour effectuer un procédé suivant l'une des revendications 1 à 16, dans laquelle l'installation-RM comprend des moyens de détection d'un signal de mouvement-d'examen pour la détermination d'une information de probabilité de mouvement et pour le réglage de la séquence d'impulsions, dans lequel, si l'installation RM est constituée pour effectuer un procédé suivant l'une des revendications 10 à 14 ou suivant l'une des revendications 15 ou 16, dans le cas où celles-ci dépendent de l'une des revendications 10 à 14, l'installation-RM comprend en outre des moyens de détection d'un signal de mouvementd'apprentissage.

18. Produit de programme d'ordinateur, qui comprend un programme et qui peut être chargé directement dans une mémoire d'une unité informatique programmable d'une installation-RM, comprenant des moyens de programme, qui sont constitués pour effectuer un procédé suivant l'une des revendications 1 à 16, lorsque le programme est réalisé dans l'unité informatique, dans lequel l'installation-RM comprend des moyens de détection d'un signal de mouvement-d'examen, pour la détermination d'une information de probabilité de mouvement et pour le réglage de la séquence d'impulsions, dans lequel, si les moyens de programme sont constitués pour effectuer un procédé suivant l'une des revendications 10 à 14 ou suivant l'une des revendications 15 ou 16, si celles-ci dépendent de l'une des revendications 10 à 14, l'installation-RM comprend en outre des moyens de détection d'un signal de mouvement-d'apprentissage.
